# EUROPEAN PATENT APPLICATION

(11) **EP 0 793 967 A2**
(43) Date of publication of application: **10.09.1997**
(21) Application number: 97103421.0
(22) Date of filing: 03.03.1997
(51) Int. Cl.: A61K 47/48

(54) **Conjugated compounds of bile acids with thiotaurine and its N-alkyl derivatives**

(30) Priority: 04.03.1996 IT MI960413
(71) Applicant: PRODOTTI CHIMICI E ALIMENTARI SPA, 15060 Basaluzzo (Alessandria) (IT)
(72) Inventor: Parenti, Massimo, c/o Prod.Chim.E Alim. SPA, 15060 Basaluzzo (IT)
(74) Representative: Michelotti, Giuliano

(57) **Abstract**

Compounds having the formula: 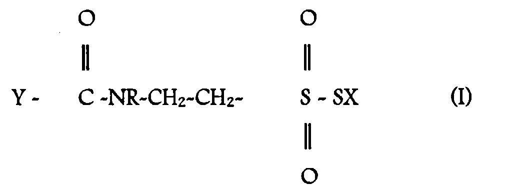 in which Y represents the residue of a bile acid, R represents H or a C₁-C₄ alkyl group, and X represents H, Na, K, Li, ½Mg, ½Ca, or a radical of a basic amino-acid or of choline are described.

The method of synthesizing them is also described.

## Description

The present invention relates to new compounds with pharmacological activity resulting from conjugation between bile acids and thiotaurine and N-alkyl derivatives thereof.

Bile acids such as ursodeoxycholic acid (UDCA), chenodeoxycholic acid (CDCA), cholic acid, deoxycholic acid, iocholic acid and iodeoxycholic acid are known for their therapeutic properties and have now been used for many years for the treatment of disorders in the area of the liver.

Ursodeoxycholic acid in particular, the structure of which is given below: 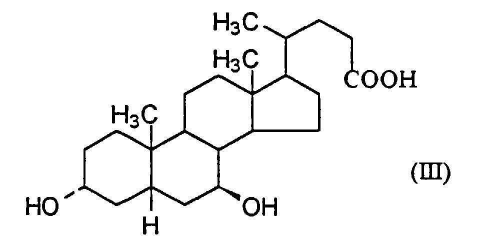 is used widely in the treatment of liver dysfunctions since it acts both by attenuating the toxic effect of some bile salts such as, for example, lithocholate on the metabolic pathways, and by decreasing mitochondrial damage, diluting the toxic bile salts by hypercholeresis, and increasing the immunomodulating effect of the bile acids. Like UDCA, its derivatives conjugated with taurine are commonly used for the treatment of liver diseases.

Italian patent No. 1167038 describes a method of synthesizing tauro-ursodeoxycholic acid, shown below: 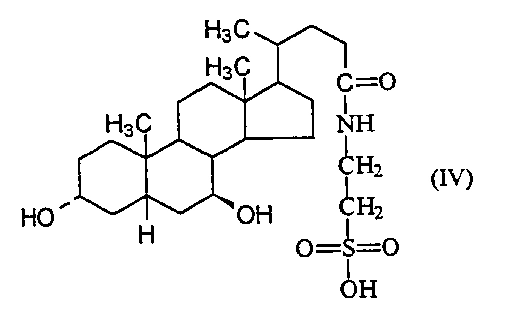 based on the use of ethylethoxycarbonyl-1,2-dihydroquinoline (EEDQ) and isobutyl-isobutoxycarbonyl-1,2-dihydroquinoline (IIDQ) as coupling agents, whilst Italian patent No. 1197330 describes a method of producing and crystallizing tauro-ursodeoxycholic acid in di-hydrate form.

United States patent No. 5,260,462, on the other hand, refers to a method of purifying tauro-ursodeoxycholic acid di-hydrate with the use of ion-exchange resin.

Italian patent application No. MI91A001059 describes N-alkyl derivatives of TUDCA which, as well as having the same pharmacological properties as TUDCA, are also resistant to deconjugation induced by the bacteria present in the colon, which may lead to the formation of toxic bile acids.

A novel class of compounds which constitute the subject of the present invention and have the structure given below has now been synthesized: 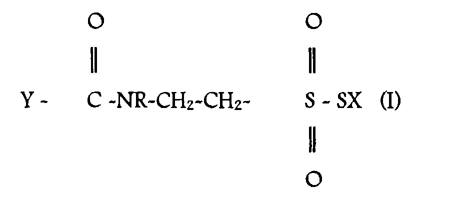 in which:
Y represents the residue of ursodeoxycholic, chenodeoxycholic, cholic, deoxycholic, iocholic, or iodeoxycholic acid;
R represents H or a C₁-C₄ alkyl group;
X represents H, Na, K, Li, ½Mg, ½Ca, or a radical of a basic amino-acid such as, for example, lysine, arginine, ornithine and histidine, or of choline.

These compounds can effectively stimulate homeostasis of the liver cells and re-establish the functional capacity of the hepatocytes, acting as enzyme promoters; they can also improve the biochemical parameters of the liver and have a considerable therapeutic effect in chronic cholesteric hepatopathy such as primitive biliary cirrhosis, sclerosing colongitis, chronic hepatitis and cirrhosis of the liver. The compounds of the present invention are also particularly important drugs for dissolving gallstones and lowering the cholesterol content of body fluids.

Although this should not be interpreted as a limitation of the invention, it seems plausible that these considerable advantages are probably due to the simultaneous presence of the -SO₂ group and of the -SH group; -SH groups in fact perform a function of preventing, delaying and inhibiting lipoperoxidation of the mitochondria and of the microsomes, which are potent inducers towards those enzymes which contain -SH groups, such as, for example, glutathione, coenzyme A, hexokinase, L.D.H., and succinic acid dehydrogenase.

Another property of these novel compounds which should not be overlooked is that, since they are not molecules which are present in the group of mammalian bile acids, they should probably be resistant to bacterial deconjugation whilst being recognized as physiological conjugated bile acids. For this reason, the compounds of the present invention can perform their therapeutic functions more effectively and for a clearly longer period of time than the conjugated bile acids which are commonly present in mammalian bile but which are deconjugated once they have reached the intestine.

Amongst the novel compounds of the present invention, those shown below 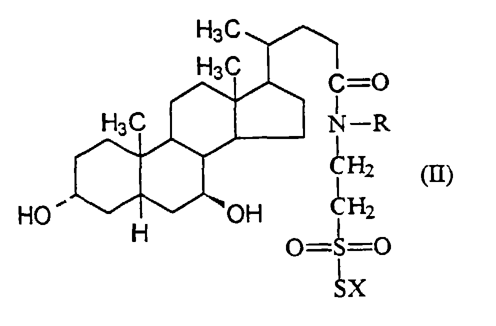 are of particular importance (the meanings of X and R are the same as those described above), that is, those in which Y is represented by ursodeoxycholic acid; the chemical names of the non-dissociated forms of compounds (II) are:
- 2-[[3a-7b-dihydroxy-24-oxo-5b-cholan-24-yl]amino]-ethanethiosulphonic acid, in the case where R = H;
- 2[[[3a-7b-dihydroxy-24-oxo-5b-cholan-24-yl]alkylamino]-amino]-ethanthiosulphonic acid, if R = C₁-C₄ alkyl.

Further subjects of the present invention are represented by pharmaceutical compositions containing the novel compounds and by the synthesis method for producing them.

The preparation of the compounds of the present invention is characterized by the following steps:
a) preparation of the mixed anhydride of the bile acid with an alkylhalogen carbonate having the structure given below: 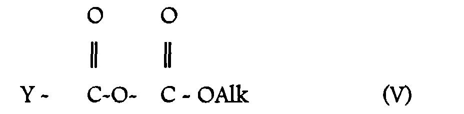 in which Alk represents a C₁-C₄ alkyl radical and the meaning of Y is the same as that described above;
b) reaction of the mixed anhydride with the compound given below:

   HRN-CH₂-CH₂-SO₂SX (VI)

   in which the meanings of R and X are the same as those described above;
c) purification and isolation of the compounds thus obtained.

The following examples are provided purely by way of non-limiting illustration of the invention.

### EXAMPLE 1

### a) mixed anhydride of ursodeoxycholic acid

14g of ethyl chloroformate were added dropwise over 15-30 minutes with stirring to a suspension of 50g of UDCA and 13g of triethylamine in 300ml of acetone cooled to - 10°C, the temperature being kept between - 10 and -5°C. Upon completion of the addition, the suspension was stirred for a further 15-30 minutes and its temperature was allowed to rise to ambient temperature. The suspension was then filtered, thus separating the triethylamine hydrochloride from the organic solution containing the mixed anhydride of UDCA which was used as it was for the subsequent reaction.

### b) sodium salt of thiotauro-ursodeoxycholic acid

A solution of 18g of thiotaurine (2-amino-ethanthiosulphonic acid) in 127 ml of 1N NaOH was prepared.

The solution of the mixed anhydride obtained in step a) was poured into this solution; the solution thus formed was stirred at ambient temperature for 3 hours and was then filtered to remove the unreacted thiotaurine.

### c) Separation and filtration of the sodium salt of thiotauro-ursodeoxycholic acid

The solution from step b) was concentrated to dryness under vacuum at a maximum temperature of 50°C. 200 ml of ethanol was added to the residue thus obtained; stirring was continued for one hour, after which the mixture was filtered. The solution thus obtained was concentrated to a small volume under vacuum at a maximum temperature of 50°C; the concentrated solution was then added to 2 litres of cold acetone and kept overnight with stirring; after filtration, the product thus obtained was dried at 50°C under vacuum, giving 40g of sodium thiotauro-ursodeoxycholate.

| Elementary analysis: | | | |
|---|---|---|---|
| • theoretical: | C: 58%; | H: 8.24%; | N: 2.6%; |
| • experimental: | C: 57%; | H: 8.6%; | N: 2.4%. |

With regard to the pharmaceutical compositions of the present invention, they are characterized in that they contain, as the active ingredient, a compound of formula I or II, together with the usual excipients and vehicles. In particular, pharmaceutical compositions containing conjugates of bile acids, and particularly of ursodeoxycholic acid, with taurine may be taken as terms of reference for the compositions of the present invention.

Similar reference may be made with regard to dosage and posology.

## Claims

1. Compounds of the formula: 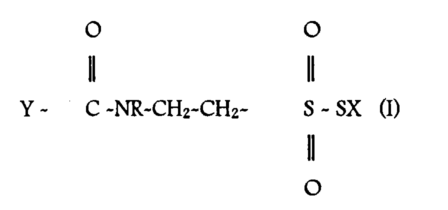 in which:
Y represents the residue of ursodeoxycholic, chenodeoxycholic, cholic, deoxycholic, iocholic, or iodeoxycholic acid;
R represents H or a C₁-C₄ alkyl group;
X represents H, Na, K, Li, ½Mg, ½Ca, or a radical of a basic amino-acid or of choline.

2. Compounds according to Claim 1, in which the basic amino-acid is selected from lysine, arginine, ornithine and histidine.

3. Compounds according to Claim 1 in which Y is the residue of ursodeoxycholic acid.

4. Compounds according to Claim 1 of the formula: 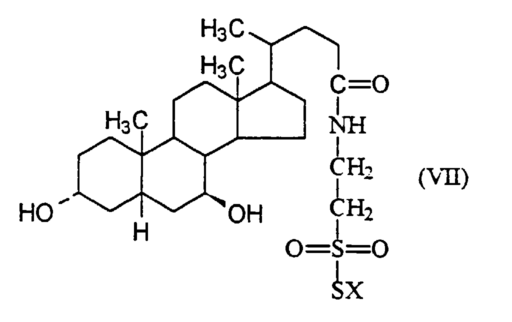 in which X has the meaning indicated in Claim 1.

5. Pharmaceutical composition, characterized in that it contains, as an active compound, a compound according to Claim 1, together with the usual excipients and vehicles.

6. Use of a compound according to Claim 1 as a medicinal substance in the treatment of hepatopathy, hepatitis, cirrhosis of the liver and gallstones.

7. Method of preparing compounds according to Claim 1, characterized by the following steps:
a) preparation of the mixed anhydride of the bile acid with an alkyl halogen carbonate having the structure: 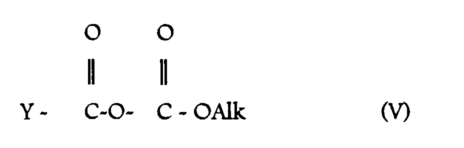 in which Alk represents a C₁-C₄ alkyl radical and Y represents the residue of ursodeoxycholic, chenodeoxycholic, cholic, deoxycholic, iocholic, or iodeoxycholic acid;
b) reaction of the mixed anhydride with the compound:
HRN-CH₂-CH₂-SO₂SX (VI)
in which R = H, or a C₁-C₄ alkyl group, and X has the meaning given in Claim 1;
c) purification and isolation of the compound thus obtained.

8. Method according to Claim 7, in which the bile acid is ursodeoxycholic acid.

9. Method according to Claim 7, in which the alkyl halogen carbonate is ethylchloroformate.

10. Method according to Claim 7, in which X is sodium.
